# EUROPEAN PATENT APPLICATION

(11) **EP 4 393 590 A1**
(43) Date of publication of application: **03.07.2024**
(21) Application number: 22383326.0
(22) Date of filing: 30.12.2022
(51) Int. Cl.: B01L 3/00, C08L 1/00, C09B 61/00, C09D 7/40, G01N 33/543

(54) **CELLULOSE AND NANOCELLULOSE BASED BIOINKS**

(71) Applicant: Tecnalia Research and Innovation, 20009 Donostia-San Sebastian (ES)
(72) Inventor: BIJELIC, Goran, 20009 Donostia-San Sebastian (ES); BRIZ ICETA, Nerea, 20009 Donostia-San Sebastian (ES); FERNANDEZ SAN ARGIMIRO, Francisco Javier, 20009 Donostia-San Sebastian (ES); OLALDE GRAELLS, Beatriz, 20009 Donostia-San Sebastian (ES); TEJADO ETAYO, Álvaro, 20009 Donostia-San Sebastian (ES)
(74) Representative: Hoffmann Eitle

(57) **Abstract**

The present invention relates to cellulose and nanocellulose based bioinks and the methodology for production of laminar flow devices and electrodes for advanced control of fluidic components for biosensing applications.

## Description

### Technical field

Cellulose and nanocellulose based bioinks and methodology for production of laminar flow devices for advanced control of fluidic components for biosensing applications.

### Background art

Biosensing applications or biosensors determine the presence or concentration of biological molecules, biological structures, microorganisms, etc. The term biosensor is used for a compact device that includes the biological sensitive element like antibodies, aptamers, enzymes etc. This element functions as a transducer to measure one or more analytes.

Many times, these analytes are human samples such as blood, urine, serum etc. Hence, a microfluidic system is required for sample preparations, processing the throughput, mixing the reagents and controlling the flow conditions for detection purpose. Obtaining a precise control of fluid in these types of systems is necessary and beneficial to operate such systems, moreover if an automatic control is required.

The present invention provides cellulose and nanocellulose based bioinks and the methodology for production of laminar flow devices for advanced control of fluidic components for biosensing applications.

### Description of embodiments

The present invention relates to cellulose and nanocellulose based bioinks and the methodology for production of devices such as disposable *in vitro* diagnostic devices in particular microfluidic lines and electrochemical sensors, for advanced control of fluidic components for biosensing applications.

In particular, the invention relates to a **N**ano **F**ibrillated **C**ellulose (NFC) based ink or nanocellulose based ink for the development of applications in biosensing. Nano fibrillated cellulose is currently manufactured from a number of different cellulosic sources. Wood is obviously the most important industrial source of cellulosic fibres and is thus the main raw material used to produce NFC. Bleached Kraft pulp is most often used as a starting material for NFC production followed by bleached sulphite pulp. NFC is manufactured from a pulp suspension mainly using a mechanical treatment. The authors of the present invention have developed an "ink" from standard NFC with hydrophilic properties that can be used for controlling the fluid (in capillarity flows) in printed channels, wherein by adjusting the viscosity (solvent is water) several applications can be obtained. This "ink" is a NFC or nanocellulose based ink having a viscosity of between 10 and 100 Pa·s at 100 s-1 composed of cellulose molecules forming particles of various size and shape where the average diameter of these molecules is from about 1 nm to about 1000 nm, such as from about 10 nm to about 500 nm or 50 nm to about 100 nm, and is dispensed or dissolved within a liquid/solvent (preferably water) known to the state of the art forming 0.1% to 3% mass percentage of solid content (number of grams in 100g of the liquid/sovent)). Where the nanocellulose can be optionally blended with one or multiple components in order to form a mixture that conditions the ink printability, wettability, chemical and biological function.

It is noted that techniques that are commonly used to determine NFC particle size are scanning electron microscopy (SEM), transmission electron microscopy (TEM) and/or atomic force microsocopy (AFM).

Some none limiting examples of applicability of the NFC or nanocellulose ink of the present invention (as defined above) can be selected from any of the following:
A. The cellulose bio-ink based concept of the present invention can be used for providing printed microfluidic devices mainly printed diagnostic devices. Instead of standard 3D microfluidic channels, pipes, valves, ... or paper based laminar flow assay devices that are dependent on paper quality, here we propose a concept where printed structures of cellulose-based bio-ink allow advanced control of fluid movement in both quantity and flow rate providing control of solvent and reagents flow from reservoirs to reaction chambers via fluidic channels where all of the named structures are formed by printing cellulose based bio-ink. The bioink of the present invention can be printed on paper, glass or polymer substrates.
B. Improving performance of existing microfluidic devices. The bioink of the present invention can be highly humectant, so adding a layer of the bioink into the inner part of microfluidics channels (of existing devices) will improve drastically reproducibility of wetting and control of the flow rate.
C. Surface modification of various non-cellulose substrates such as glass and polymers with covalently bound cellulose particles, where in the case of transparent substrates optical properties are minimally affected and wetting properties are similar to paper substrates due to cellulose particles introduced modification . The bioink can provide one layer of coating to any substrate (plastic, glass metal,...) giving paper properties to the substrate such as wettability.
D. Biofunctionalized ink. The bioink can be blended with bio-active agents such as antibodies or enzymes or the cellulose particles of the bioink can be decorated with physically deposited or chemically grafted bioactive agents and molecules) to provide different inks expanding current solutions in the market, for instance, printing specific areas in lateral flow immunoassays.

In this sense, and as a further non-limiting example of an application of the bioink of the present invention, the bioink can be printed over polystyrene to design 2D microfluidic circuits.

Hence, the NFC or nanocellulose ink of the present invention is thus particularly useful for providing any type of devices comprising printed structures of cellulose-based bio-ink, preferably any type of device having printed structures of cellulose-based bio-ink such as microfluidic devices including electrodes or any other device for actuation and sensing. In this sense, the present invention provides a device comprising NFC or nanocellulose ink deposited through printing processes known to the state of the art onto a solid rigid or flexible substrate (preferably a substrate suitable for laminar capillary flow devices or electrodes), where the ink once deposited onto the substrate is cured to form a device of fluid conducting lines, where such device is preferably capable of controlling the amount and the flow rate of a fluid transported through the said conducting lines. It is noted that the amount and flow rate of a fluid is mainly controlled in the present invention by the printing process, which is preferably screen printing, and the curing process in consecutive steps. Other factors such as the chemical composition of the bioink and functionalization of the substrate and/or bioink also influence the amount and flow rate of the fluid as taught in the examples described in the present specification.

Preferably, the NFC or nanocellulose ink of the present invention is deposited by a process call screen printing (a printing technique where a mesh is used to transfer the ink onto a substrate, except in areas made impermeable to the ink by a blocking stencil) or by electrodeposition.

In the present invention, as already indicated, the NFC or nanocellulose ink has a viscosity of between 10 and 100 Pa·s at 100 s-1 and is composed of cellulose molecules forming particles of various size and shapes where the average diameter of these molecules is from about 1 nm to about 1000 nm, such as from about 10 nm to about 500 nm or 50 nm to about 100 nm, and is dispensed or dissolved within a liquid/solvent (preferably water) known to the state of the art forming 0.1% to 3% mass percentage of solid content (number of grams in 100g of the liquid/sovent)). Where the nanocellulose can be optionally blended with one or multiple components in order to form a mixture that conditions the ink printability, wettability, chemical and biological function. In particular, the nanocellulose ink of the present invention is printed/dispensed on a substrate and processed to form a film or a foam, where different processing/curing methods enable control of the fluidic properties of the fluidic channels, reservoirs and reaction chambers of the device. Where the printing and/or dispensing process can be repeated in order to form a multilayer structure, where layers may adhere to each otherto form a homogenous fluidic channel of different thicknesses or where layers may form a laminar structure where each lamina has specific fluidic properties.

Therefore, a first aspect of the invention refers to a device comprising a solid, and preferably flat, substrate surface (such as a carbon electrode) characterized by comprising printed, preferably by screen printing or electrodeposition, flow fluidic channels and preferably reaction chambers and/or reservoirs, wherein said printed laminar flow fluidic channels and preferably reaction chambers and/or reservoirs are printed with a NFC or nanocellulose ink comprising a dispersion of cellulose nanofibrils in a liquid media, such as water, wherein the cellulose nanofibrils have an average diameter of from about 1 nm to about 1000 nm, such as from about 10 nm to about 500 nm or 50 nm to about 100 nm, and are dispersed or dissolved within the liquid media forming 0.1% to 3% percentage of solids by weight (this amount is determined by dividing the mass of solids by the total mass of the solution, and then multiply the result by 100 to calculate the percentage of solids by weight), and wherein the nanocellulose ink has a viscosity of between 10 and 100 Pa·s at 100 s-1. Preferably, the nanocellulose ink is blended with one or more binders wherein the binder is preferably a carboxy methyl cellulose, preferably when the nanocellulose ink comprises unmodified NFC.

In a preferred embodiment, the cellulose nanofibrils can be unmodified NFCs or modified NFCs selected from the group consisting of dicarboxylated nanofibrillar cellulose (DC-NFC), dialdehydated nanofibrillar cellulose (DA-NFC), thiolated nanofibrillar cellulose (SH-NFC), ciclooctinilated nanofibrillar cellulose (DIBCO-NFC) and aminated nanofibrillar cellulose (NH2-NFC). Preferably, the cellulose nanofibrils are covalently bounded to biological agents such as peptides, proteins, enzymes, antibodies, or nucleotide sequences to provide for a chemical and/or biological function. For this purpose, the cellulose nanofibrils have a surface that is closely packed with hydroxyl groups, which allows for chemical modifications to be performed on their surfaces.

In another preferred embodiment, the solid substrate surface is made of silicon, silicon dioxide, silicon nitride, glass, diamond, quartz, magnesium fluoride (MgF2), calcium fluoride (CaF2), ZnSe, germanium, or a polymer such as polystyrene or cellulose fibres (such as paper). Preferably, the solid substrate surface is flat, non-porous or porous, and optionally patterned. More preferably, the solid substrate surface is chemically modified to provide thiolated (SH), carboxylated (COOH), cholorsulfonated (SO2Cl-), aminated (NH2), alkenylated (alkenylated), and epoxidated (epoxydated) substrates.

It is noted that, in a preferred embodiment, the device after the flow fluidic channels, and optionally the reaction chambers and/or reservoirs, are printed with the nanocellulose ink, is further characterized by being cured with any useful technique, such as drying, freezing or heating or any combination thereof. Preferably, the device is cure by being dried (preferably oven dried at about 37ºC) or freeze dried. Preferably, the device is further characterized by being covered with NaCl in a suitable medium as described in the examples.

A second aspect of the invention refers to a method to produce a device as defined in the first aspect of the invention comprising a solid substrate surface, wherein the method comprises printing flow fluidic channels and preferably reaction chambers and/or reservoirs, preferably by screen printing or electrodeposition, onto the solid substrate surface, wherein said printed laminar flow fluidic channels and preferably reaction chambers and/or reservoirs are printed with a nanocellulose ink comprising a dispersion of cellulose nanofibrils in a liquid media, wherein the cellulose nanofibrils have an average diameter of from about 1 nm to about 1000 nm, such as from about 10 nm to about 500 nm or 50 nm to about 100 nm, and are dispersed or dissolved within the liquid media forming 0,1% to 3% percentage of solids by weight, and wherein the nanocellulose ink has a viscosity of between 10 and 100 Pa·s at 100 s-1. Preferably, the nanocellulose ink is blended with one or more binders wherein the binder is preferably a carboxy methyl cellulose, preferably when the nanocellulose ink comprises or is made of unmodified NFC.

In a preferred embodiment, the cellulose nanofibrils can be unmodified NFCs or modified NFCs selected from the group consisting of NFC, dicarboxylated nanofibrillar cellulose (DC-NFC), dialdehydated nanofibrillar cellulose (DA-NFC), thiolated nanofibrillar cellulose (SH-NFC), ciclooctinilated nanofibrillar cellulose (DIBCO-NFC) and aminated nanofibrillar cellulose (NH2-NFC). Preferably, the cellulose nanofibrils are covalently bounded to biological agents such as peptides, proteins, enzymes, antibodies, or nucleotide sequences to provide for a chemical and/or biological function.

In another preferred embodiment, the solid substrate surface is made of silicon, silicon dioxide, silicon nitride, glass, diamond, quartz, magnesium fluoride (MgF2), calcium fluoride (CaF2), ZnSe, germanium, or a polymer such as polystyrene or cellulose fibers (such as paper). Preferably, the solid substrate surface is flat, nonporous or porous, and optionally patterned. More preferably, the solid substrate surface is chemically modified to provide thiolated (SH); carboxylated (COOH); cholorsulfonated (SO2Cl-); aminated (NH2); alkenylated (alkenylated); and epoxidated (epoxydated) substrates.

It is noted that, in another preferred embodiment of the second aspect of the invention, the method is characterized by comprising a curing step after the flow fluidic channels and reaction chambers are printed with the nanocellulose ink. Said curing method can be any useful technique, such as drying, freezing or heating or any combination thereof. Preferably, the device is cure by being dried (preferably oven dried at about 37ºC) or freeze dried. Preferably, the previous step is followed by the step of covering the resultant device with NaCl in a suitable medium as described in the examples.

A third aspect of the invention refers to a device, such as microfluidic devices, microtiter plates, microarrays, or more preferably laminar capillary flow devices or electrodes, obtained or obtainable by the method of the second aspect of the invention.

A fourth aspect of the invention refers to the in vitro use of the device, preferably the laminar capillary flow device or electrode, as defined in any of first or third aspects of the invention, for biosensing applications. Preferably, for lateral flow immunoassays or for electrochemical assays such as enzymatic detection.

In particular, the devices of the present invention can be use in at least three application fields: In vitro diagnostics (disposable in vitro diagnostic devices in particular microfluidic lines and electrochemical sensors), biomedical electrodes (for detection or initiation of electrophysiological event and transdermal delivery of active substances or biomarker extraction), functional consumables (plasters, bandages and bed covers). The scope of invention is addressing industrial scale production of cellulose based biomedical sensors and advanced medical consumables and devices through additive manufacturing process. The technology platform and pilot lines primary target is in sectors of In vitro diagnostics with paper based microfluidic lines and bio-functionalised electrochemical sensors, biomedical electrodes for the detection or initiation of electrophysiological event and transdermal delivery of active substances or biomarker extraction, functional consumables such as "intelligent" electronic plasters, bandages and bed covers.

Lastly, it is herein indicated that the printing technology indicated herein can be, and is included in the present invention, adapted to industrial scale such as screen printing, inkjet printing, AerosolJet^{®} printing, roller based printing, filling of roller based imprinted structures and 3D printing.

### Brief description of the figures

**Fig. 1****.** Bio-ink fluidic device where two identical structures are shown where each comprises 1 reaction chamber, 3 reservoirs connected by 3 different patterns of linear fluidic channels. Each of the channel patterns differs in number of channel lines and their thickness, allowing control of fluid flow.
**Fig. 2****.** Structures designed to study fluidic control.
**Fig. 3****.** shows wettability and consequent printability of NCF on different PS substrate modifications compared with water.
**Fig. 4****.** shows wettability and consequent printability of 3 different NCF inks on PS and selected modified PS substrates.
**Fig. 5****.** Screening of printable capacity of different inks on different surfaces.
**Fig. 6****.** Graph derived from the study of the rheology of the nanocellulose to determine the viscosity of the ink.
**Fig. 7****.** Contact angle test on different functionalized polystyrenes, using water and ink ref.C. The evolution of the drop is measured for 30 seconds.
**Fig. 8****.** Contact angle test on different functionalized polystyrenes, using water and NFC ink ref. C. The evolution of the drop is measured for 30 seconds.
**Fig. 9****.** Study of the relation between the concentration of NFC printed and the flow rate. After the printing of NFC bioink on selected surfaces (aminated polystyrene) they were freeze dried and the flow velocity was measured as shown in the figure.
**Fig. 10****.** EIS results from the different electrodes analysed in fresh, after 3 months of incubation at 4 °C and after 3 months of incubation at room temperature (RT).
**Fig. 11****.** This figure shows how none of the concentrations tested of the NFC unmodified as shown in the examples were considered toxic. All viabilities were above 80%.

The following examples are merely illustrative of the present invention and do not limit the same.

### Examples

### NANOCELLULOSE

Toxicity of a nanocellulose fibril (NCF) sample, unmodified NFC, with a vegetable origin was evaluated under the ISO 10993-5 standard test. Samples were either dialyzed (dyalisis) or not (reference). The aim of this test is to evaluate biologically, the toxicity of the samples by lysis of the cells, the inhibition of cell growth, and other effects on cells, caused by the materials or their extracts.

Briefly, 10⁴ cells were seeded on each well and left for 24h at 37ºC in an incubator (5% CO2). Then, NFC was dispersed in cell culture media and different concentrations were prepared. 100µl of each concentration (2000µg/ml; 1000µg/ml; 500µg/ml; 100µg/ml; 50µg/ml) were added to the each well (n=6). Results are based on the quantitative evaluation of the cell viability after 24 hours of the sample contact. Cell viability is quantified by means of the WST-1 test. This is a colorimetric method that detects mitochondrial activity in living cells. The tetrazolium salts are cleaved by cellular enzymes to produce the formazan. The increased number of viable cells is an increase in the activity of mitochondrial dehydrogenases in the sample, and an increase in the amount of formazan formed, which is directly proportional to the number of metabolically active cells in the culture. Thus, after sample extracts' treatment, WST-1 reagent is added to each well and 4 hours later, the optical density of this solution in each well is measured at a wavelength of 450 nm using a microplate reader (Biotek, Powerwave XS). According to the ISO 10993-5, if cell viability at the highest concentration analyzed (named 100%) is below 70%, the sample is considered toxic.

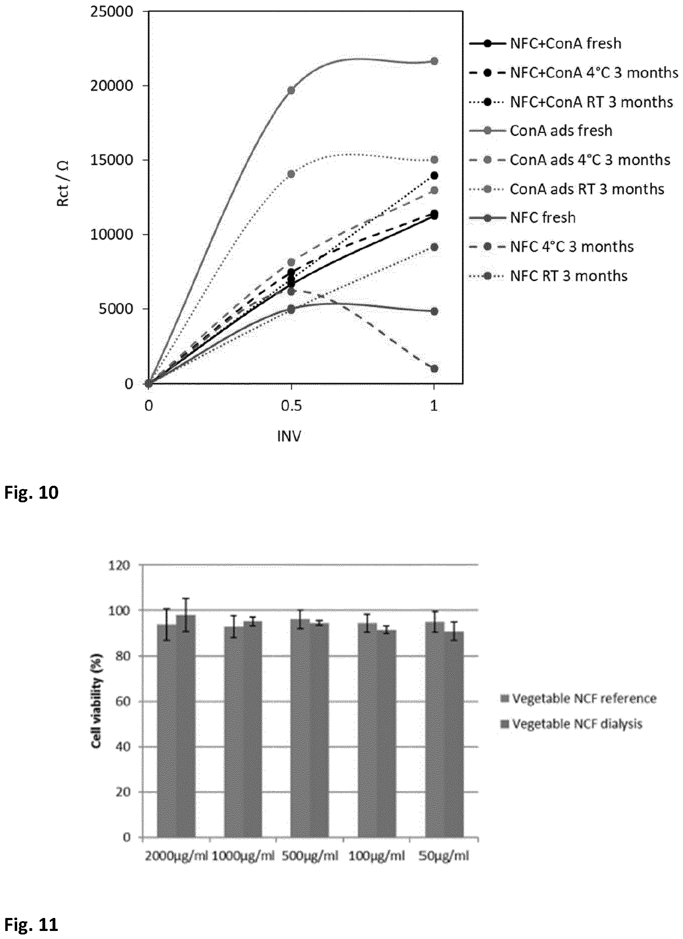

**Results can be seen in** **figure 11****. This figure shows how none of the concentrations tested** were considered toxic. **All** viabilities were above 80%.

### FUNCTIONALIZED NANOCELLULOSE

### Chemical functionalization of nanocellulose

Different chemical modifications of nanocellulose were study from the original Nanofibrillar cellulose (NFC), obtaining dicarboxylated nanofibrillar cellulose (DC-NFC), dialdehydated nanofibrillar cellulose (DA-NFC), thiolated nanofibrillar cellulose (SH-NFC), ciclooctinilated nanofibrillar cellulose (DIBCO-NFC) and aminated nanofibrillar cellulose (NH2-NFC).

Dicarboxylated nanofibrillar cellulose (DC-NFC) and dialdehydated nanofibrillar cellulose (DA-NFC) are the result of the oxidation of the NFC, being DA-NFC the product of the partial oxidation of the NFC and DC-NFC is the product of the complete oxidation of the NFC.

The thiolation of NFC was performed studying the functionalization of DC-NFC and DA-NFC with cystamine:

### - Thiolation of DA-NFC

Dissolution of DA-NFC was reacted with cystamine in a sodium carbonate and sodium citrate buffer at pH 9.5 and sodium cyanoborohydride was added as reducing agent (scheme 1). The reaction was 2h at RT. Then the reaction was dialyzed in NaCl saturated dissolution and water. Finally, the reacted DA-NFC was washed with water by centrifugation.

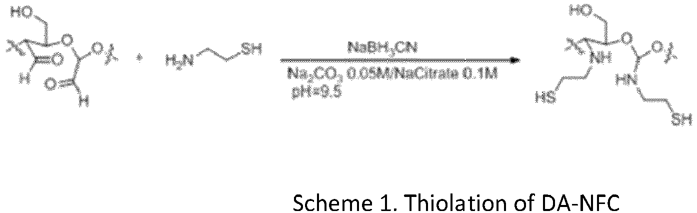

### - Thiolation of DC-NFC

A method for activating carboxylic groups is to use EDC/NHS reactants.There are two ways to make this reaction, one step (scheme 2) or two steps (scheme 3).

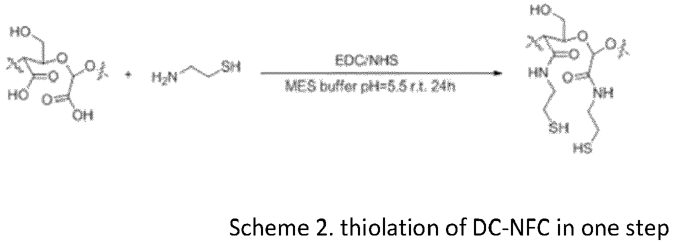

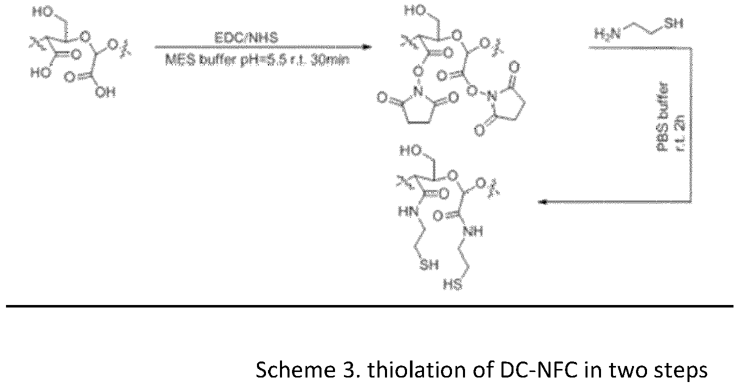

The characterization of the thiol groups introduced in NFC was carried out by colorimetric assay using Ellman's reagent, confirming that the most efficient thiolation was observed on DA-NFC (table 1):

**Table 1. thiol groups quantification in cystamine modified NFC.**

| | **nmol SH/mg NFC** |
|---|---|
| **Cys-DA-NFC** | 1,78 |
| **Cys-DC-NFC one step** | 0,45 |
| **Cys-DC-NFC 2 steps** | 0,08 |

The amination of NFC was performed on DA-NFC using ethylendiamine saturated solution to avoid second intramolecular amidation (scheme 4).

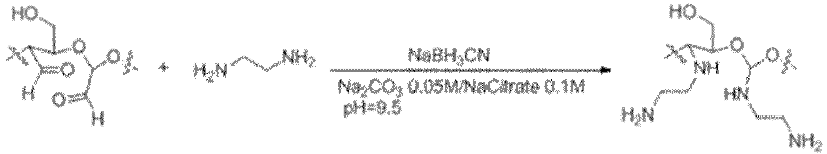

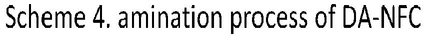

### - DIBCO modification of NFC was performed on DA-NFC using dibenzocycloktine amine (DIBCO-NH2) (scheme 5)

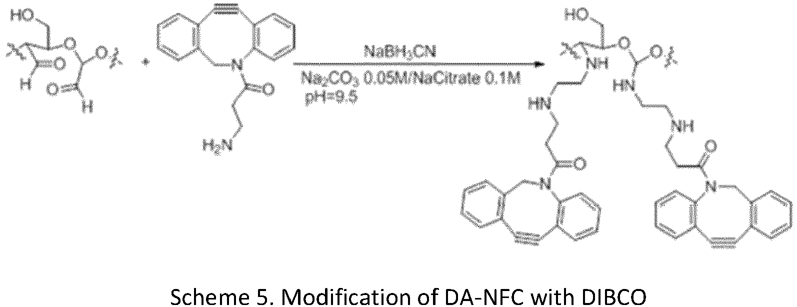

To characterize the quantity of alkyne groups presents on the NFC click chemistry reaction with 7-(Diethylamino) coumarin-3-carbonyl azide was performed. The quantity of 7-(Diethylamino) coumarin-3-carbonyl azide attached could be observed by fluorescence and determined using a standar curve. As a control nonmodified DA-NFC was evaluated. The result was 15.18 nmol of alkyne groups/mg NFC.

The next table (table 2) summarizes the different modified NFC developed for the present invention and their characterization:

**Table 2. Different chemical modification of NFC**

| **NFC modification** | **Functional groups** | **Characterization** | **Formula** |
|---|---|---|---|
| **DC-NFC** | **COOH** | **134,26 nmol COOH/mg DC-NFC (72,51 nmol COOH/mg NFC)** | 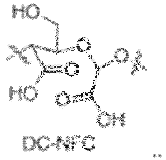 |
| **DA-NFC** | **CHO** | **FTIR missing signal of band at 910cm**^{**-**1} | 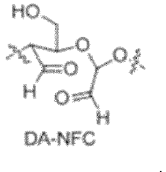 |
| **SH-NFC** | **SH** | **1.78 nmol/mg SH-DA-NFC** | 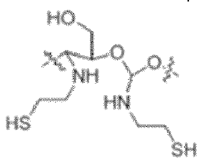 |
| | | **0.45nmol/mg SH-DC-NFC** | |
| **DISCO-NFC** | **DISCO** | **15,18nmol/mg NFC** | 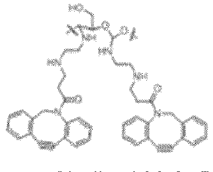 |
| **NH₂-NFC** | **NH₂** | **50pmol/mg NFC** | 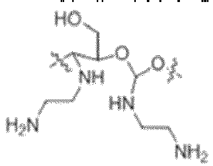 |

### Viscosity & printing

Once the bioink has been modified as indicated in the previous section, we proceeded to study the adhesion and printing of the inks. The printing technique selected was the "screen printing" technique due to its high reproducibility and because it is a technique applicable on a large scale. For the "screen printing" technique it is necessary to use inks that are pseudoplastic, that is to say that they are viscous so that they do not flow through the holes of the mesh but when a force is applied, the ink loses viscosity and behaves in a more liquid manner so that it can flow through the holes in the mesh.

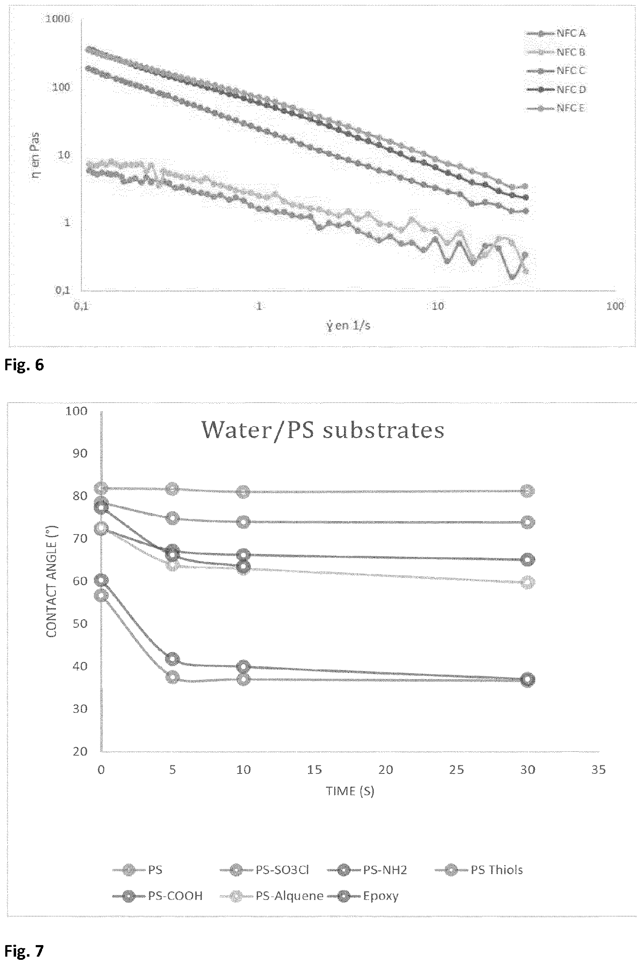

For screen printing, it is desirable that the inks have a viscosity between 10 and 200 Pa/s. The rheology of the nanocellulose was studied to see the viscosity of the ink. We use the carboxylated nanocellulose as a model, since it is the starting nanocellulose that we use in the other functionalizations. We prepare nanocellulose inks at different concentrations. From the data we obtained, we saw that the most diluted inks do not reach 10 Pa/s, they are very liquid, therefore, they are not suitable for printing. On the other hand, we saw that the most concentrated inks are very viscous, they are above 200 Pa/s. The best results were obtained for ref. C as described in figure 6. This ink behaves like a solid and does not flow through the holes in the mesh, but when we apply force to print the ink behaves like a liquid and flows through the holes of the mesh.

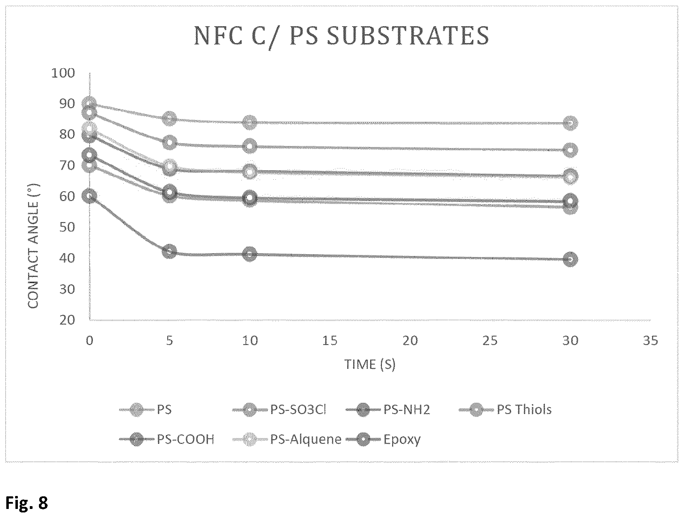

To study the surface on which the ink was going to be printed, a contact angle test was carried out on different functionalized polystyrenes, using water and ink ref.C, the evolution of the drop was measured for 30 seconds. As shown in figure 7, with a drop of water, in the samples where the contact angle with water is made, it is observed that the carboxylated and thiolated surfaces have the most hydrophilic behavior, while the polystyrene and chlorosulfonated polystyrene have the most hydrophobic behavior. When studying the contact angle with the NFC ref. C (see figure 8), it is observed that due to the functional charge and the viscosity of the NFC, the wettability of the surfaces changes in comparison with that of water, increasing the hydrophobic character in all cases.

### Printed microfluidic on different substrates (paper, polymers, textiles) for fluidic control

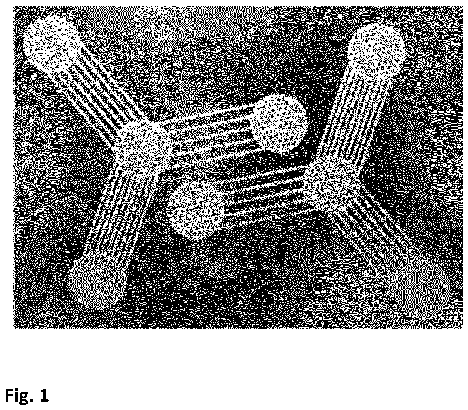

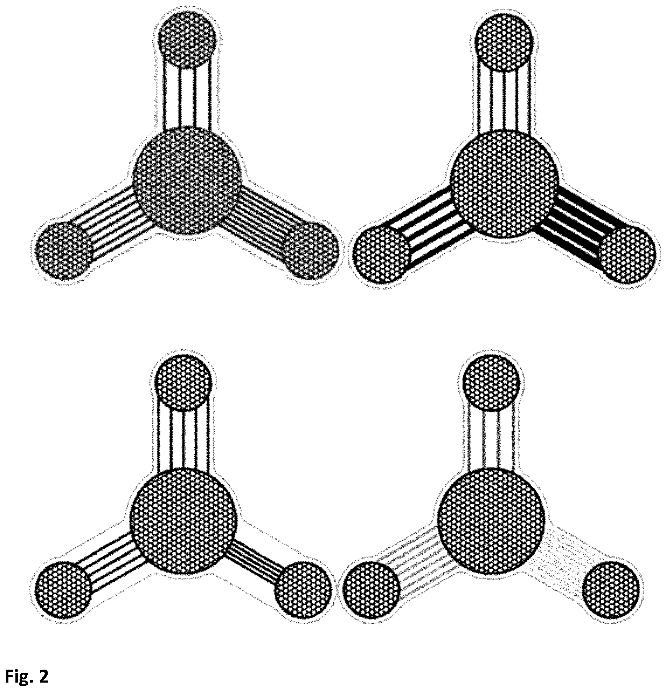

In order to investigate the efficacy of flow rate control, specific reservoir, channels and reaction chamber designs were compared. Moreover, various ink compositions and substrate surfaces were investigated. The NFC bioink was screen printed (Fig 1) on the selected design, where alternative designs are shown in (Fig 2). A battery of samples (n=50) was printed with the selected combinations to study the fluidic behavior.

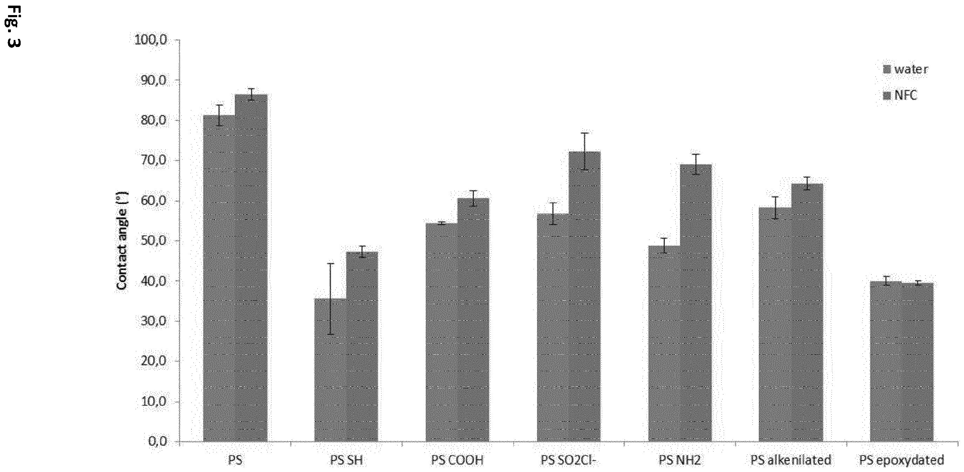

On the other hand, in order to design the Nanocellulose bioink that can be printed as described herein, basic physicochemical properties of ink to substrate interface have been studied (figure 3). For this purpose, the selected substrate was polystyrene where controlled chemical modification of polystyrene surface was done through chlorosulfonation and posterior sulfoamidation, being: PS, polystyrene; PS SH, thiolated polystyrene; PS COOH, carboxylated polystyrene; PS SO2Cl-, cholorsulfonated polystyrene; PS NH2, aminated polystyrene; PS alkenylated, alkenylated polystyrene; and PS epoxydated, epoxidated polystyrene.

Measurement of contact angle was performed, for all above named surfaces, and being a thermodynamic quantity a unique value for any ink/surface interface could be expected. However, the behaviour of bioink (NFC waterborne suspension, 0,5%), had a very similar wetting behaviour as observed for pure water in all PS surfaces, with more pronounced hydrophobicity of the NFC bioink (except on epoxylated PS). This overall similarity could have origin in hydration and hydrogen bonds NFC particles have with surrounding water molecules and the fact that 95.5% of the bioink is water. Looking at overall both, water and bioink, different grade of wetting for different PS surfaces were observed. Where the thiolated and epoxydated polystyrenes are the most hydrophilic and unmodified polystyrene together with cholorsulfonated are the most hydrophobic. Origin of this behaviour can be deduced from theoretically expected behaviour of different surface functional groups and correlated to the number of hydrogen bonds.

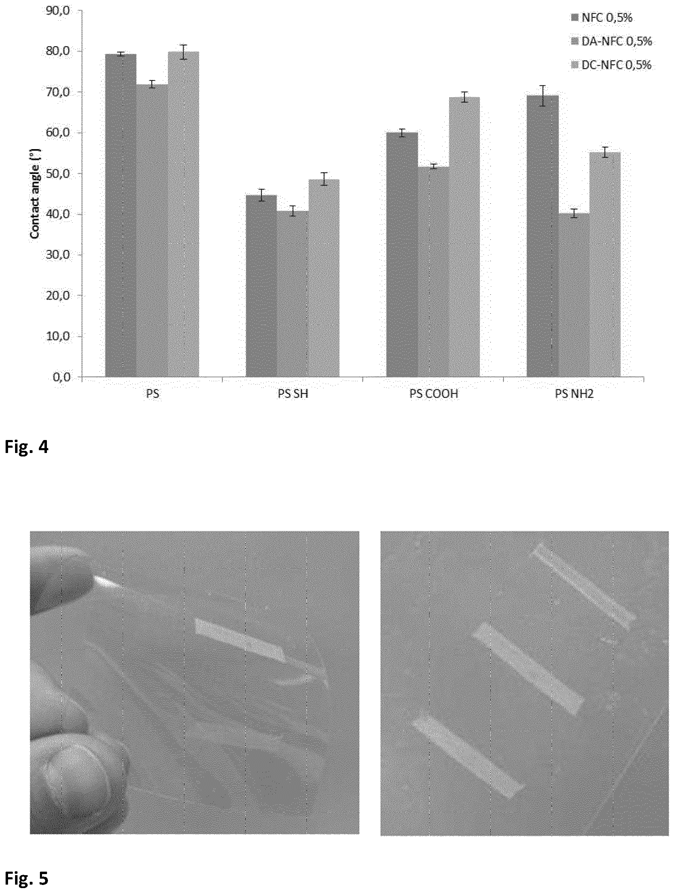

Through chemical modifications of NFC, wettability and the chemical interaction between ink and surfaces can be changed and in consequence the printing capability. Figure 4 shows the change in wettability on the surfaces of the dicarboxilated NFC (DC-NFC) and aldehydated NFC (DA-NFC). It can be observed that the bioink with grafted aldehyde to NFC has best wetting properties in all surfaces because it has more polar functional groups than nonmodified NFC. However, such behaviour is less pronounced for the carboxilated NFC that is still more polar than nonmodified NFC. The chemistry of the PS surfaces plays a role making that the contact angle of the ink increases on negatively charged surfaces (PS SH, PS COOH) and decreases on positive charged surfaces (PS NH2), that is clearly consequence of strong electrostatic interactions between particles with carboxyl groups and positively charged surfaces.

### Printing of nanocellulose

### Proof of concept

The printing capacity of nanocellulose was studied by manual deposition using a mask and removing the excess with Teflon blade (figure 5). The screening was performed on different modified polystyrene surfaces with different modified NFC. After printing the samples were cured using two methods: freeze drying and at 37ºC, then they were covered with NaCl 2M in water under stirred at 75 rpm for 2 hours. The results are shown in table 3.

**Table 3. Screeing of printable capacity of differents inks on different surfaces**

| | NFC | | NFC-COOH | | NFC-NH2 | | NFC-SH | |
|---|---|---|---|---|---|---|---|---|
| Surface/Drying process | FD | 37C | FD | 37C | FD | 37C | FD | 37C |
| PS | x | | x | | x | x | x | x |
| PS-SO2Cl | x | | x | | ok | ok | | |
| PS-NH2 | x | | ok | | | | | |
| PS-COOH | x | | | | ok | ok | | |
| PS-COOH NaOH 1M | ok | | | | x | ok | | |
| PS-COOH NHS/EDC | ok | | | | ok | ok | | |
| PS-SH | x | | | | | | ok | ok |

| | | | | | | | | |
|---|---|---|---|---|---|---|---|---|
| FD: freeze drying 37ºC: oven drying | | | | | | | | |

It could be observed that the samples oven dried stick on better to the surface; this could be because the freeze-dried samples are more porous and the interphase between the ink and the surface is smaller. Any inks stuck to PS surface because of its hydrophobicity.

### Screen printing

The screen printing of pure NFC bioink on various surfaces selected during the proof of concept showed good surface adhesion after curing, in particular, when the bioink further comprises the addition of a polymeric binding component such as carboxy methyl cellulose.

### Fluidic control

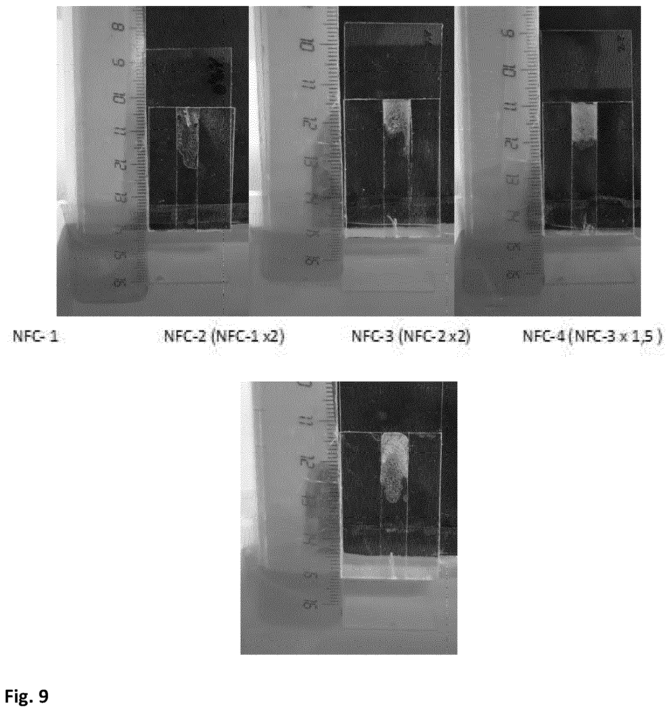

NFC printable bioink, apart from the applications related to biofunctionalization and control of wetting of microstructures (described previously), it has potential for use in the manufacturing of laminar flow fluidic channels on flat surfaces. In order to investigate the efficacy of flow rate control, specific reservoir, channels and reaction chamber designs were compared. Moreover, various ink compositions and substrate surfaces were investigated. We have investigated relation between the concentration of NFC printed and the flow rate. After the printing of NFC bioink on selected surfaces (aminated polystyrene) they were freeze dried and the flow velocity was measured (see figure 9 and table 5 below).

**Table 5.**

| Ink | Flow rate (mm/s) |
|---|---|
| | |
| NFC-1 | 1,63 |
| NFC-2 | 2,57 |
| NFC-3 | 1,15 |
| NFC-4 | 0,38 |

NFC concentration number 3 was the one selected to printing the NFC due to its best ratio between observed printability and flow rate.

### Permanent wettability of hydrophobic surfaces (application in microfluidic devices)

The filled of the channels with NFC could assure the wettability of the channels without requiring special conditions of storage. After plasma treatment of the structured surfaces the channel were filled with NFC, then surfaces were dried at 37ºC. The wettability of the channel increases significantly.

### BIOFUNCTIONALIZED NANOCELLULOSE

### Chemical biofunctionalization of nanocellulose

### Polylysine biofunctionalized nanocellulose

DA-NFC was functionalized with poly-D-lysine (PDL). DA-NFC was mixed with dissolution of PDL and the reaction mixture was stirred at 37°C for two hours. Then the resulting mixture was dialyzed with saturated dissolution of NaCl in water and water. The characterization of the PDL was performed by ADECA colorimetry assay using NFC as control. 12.76 pmol of PDL/mg DA-NFC was obtained.

### RGD biofunctionalized nanocellulose

DIBCO-NFC was modified with azide RGD peptide mimetic through click chemistry reaction (Scheme 6).

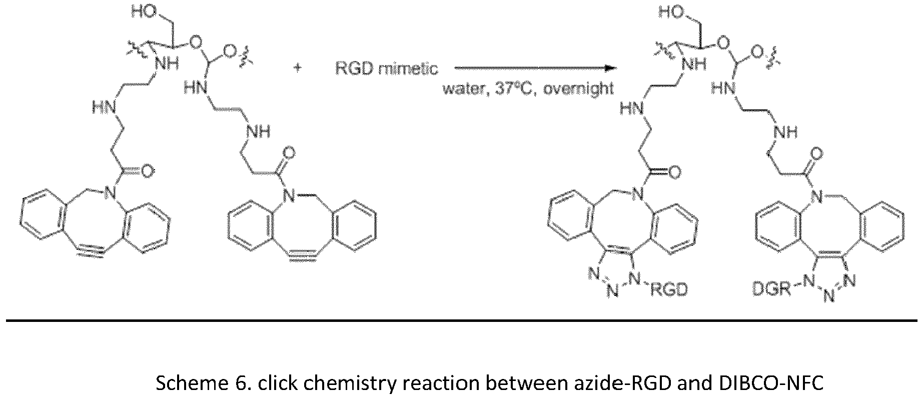

The characterization of RGD was performed by immunoassay obtaining 0,14 pmol RGD/mg NFC (SD 0,02).

### Coating of electrodes of electrochemical assays

Screen-printed carbon electrodes modified with NFC-ConA were used for the determination of the glycoprotein invertase (INV), a typical molecule utilized as a model analyte. This glycoprotein presents a high affinity towards Concanavalin A (ConA) lectin. Their interaction leads to the formation of a stable complex that can be measured by electrochemical techniques.

Impedance measurements were carried out to evaluate the response of the modified electrodes to different INV concentrations. To do this, NFC-ConA electrodes were incubated with different INV solution for 30 min at room temperature. Afterwards, they were rinsed, dried and kept in dark. For the impedance measurements we used the ferricyanide system, a redox probe ([Fe(CN6)3+/4+]). After the incubation with the INV, impedance measurements were performed in presence of [Fe(CN6)3+/4+] 5mM in PB buffer at room temperature.

Two control experiments were performed at the same time in order to get more information about the NFC role in the immobilization of the ConA:
- NFC modified electrodes: scree-printed electrodes modified only with nanocellulose.
- ConA deposited by adsorption: simple adsorption of the lectin on the surface of the electrode without NFC.

Different conditions of storing and measurement were compared in order to get a complete study of the NFC role in the stabilization con the lectin on the surface of the electrodes:
- In situ measurement after the electrodes modification.
- Measurement after storing for 3 months 579 at 4°C in dark.
- Measurement after storing for 3 months at room temperature (RT) in dark.

The results from the impedance measurements of all the systems are summarize in Figure 10. We represented the value of the impedance (Rct) with the amount of INV analysed. The impedance increases when increasing INV concentration due to the interaction between the protein and the concanavalin immobilized. The complex formed after the interaction blocks the surface of the electrode and produces and increase in the impedance signal.

From the results above mentioned we can conclude that NFC allows a better immobilization and stabilization of the ConA. The results of NFC ConA electrodes are similar before (black solid line) and after (black dashed line) the storage for 3 months, either at 4 °C (black dashed line) or at room temperature (black dotted line). On the other hand, the electrodes with ConA adsorbed (blue lines) lose the reactivity after that time under the conditions studied (decrease of the impedance values with time). Moreover, NFC controls (red lines) did not show a significant reactivity when increasing INV concentration.

We thereby demonstrated that the modification of the electrodes with nanocellulose combined with ConA offers a stable and reactive system for the determination of invertase through impedance measurements. This protocol enables the proper immobilization of the lectin with no lose in reactivity and a higher stability of the system. The modified electrodes can be easily stored under not sophisticated conditions, this is, room temperature or 4 °C.

## Claims

1. A device comprising a solid substrate surface **characterized by** comprising printed, preferably by screen printing or electrodeposition, flow fluidic channels and preferably reaction chambers and/or reservoirs, wherein said printed laminar flow fluidic channels and preferably reaction chambers and/or reservoirs are printed with a nanocellulose ink comprising a dispersion of cellulose nanofibrils in a liquid media, such as water, wherein the cellulose nanofibrils have an average diameter of from about 1 nm to about 1000 nm, such as from about 10 nm to about 500 nm or 50 nm to about 100 nm, and are dispersed or dissolved within the liquid media forming 0,1% to 3% percentage of solids by weight, and wherein the nanocellulose ink has a viscosity of between 10 and 100 Pa·s at 100 s-1.

2. The device of claim 1, wherein the cellulose nanofibrils are selected from the group consisting of dicarboxylated nanofibrillar cellulose (DC-NFC), dialdehydated nanofibrillar cellulose (DA-NFC), thiolated nanofibrillar cellulose (SH-NFC), ciclooctinilated nanofibrillar cellulose (DIBCO-NFC) and aminated nanofibrillar cellulose (NH2-NFC).

3. The device of any one of claims 1 or 2, wherein the cellulose nanofibrils are **characterized by** being covalently bounded to biological agents such as peptides, proteins, enzymes, antibodies, or nucleotide sequences to provide for a chemical and/or biological function.

4. The device of any one of claims 1 to 3, wherein the solid substrate surface is made of silicon, silicon dioxide, silicon nitride, glass, diamond, quartz, magnesium fluoride (MgF2), calcium fluoride (CaF2), ZnSe, germanium, or a polymer such as polystyrene or cellulose fibers (such as paper).

5. The device of claim 4, wherein the solid substrate surface is flat, nonporous or porous, and optionally patterned.

6. The device of any one of claims 4 or 5, wherein the solid substrate surface is chemically modified to provide thiolated (SH); carboxylated (COOH); cholorsulfonated (SO2Cl-); aminated (NH2); alkenylated (alkenylated); and epoxidated (epoxydated) substrates.

7. The device of any one of claims 1 to 6, wherein after the flow fluidic channels and reaction chambers are printed with the nanocellulose ink, the device is cured by drying, freezing or heating or any combination thereof.

8. The device according to claim 7, wherein the device is cure by being dried (preferably oven dried at about 37ºC).

9. The device according to any one of the precedent claims, wherein the nanocellulose ink is blended with one or more binders.

10. The laminar capillary flow device according to claim 7, wherein the binder is selected from the list consisting of Poly3, Poly5 and Poly6.

11. In vitro use of the laminar capillary flow device as defined in any of claims 1 to 10, for biosensing.

12. A method to produce a device comprising a solid substrate surface, wherein the method comprises screen printing flow fluidic channels and preferably reaction chambers and/or reservoirs, wherein said printed laminar flow fluidic channels and preferably reaction chambers and/or reservoirs are printed with a nanocellulose ink comprising a dispersion of cellulose nanofibrils in a liquid media, such as water, wherein the cellulose nanofibrils have an average diameter of from about 1 nm to about 1000 nm, such as from about 10 nm to about 500 nm or 50 nm to about 100 nm, and are dispersed or dissolved within the liquid media forming 0,1% to 3% percentage of solids by weight, and wherein the nanocellulose ink has a viscosity of between 10 and 100 Pa·s at 100 s-1.

13. The method of claim 12, wherein the cellulose nanofibrils are selected from the group consisting of dicarboxylated nanofibrillar cellulose (DC-NFC), dialdehydated nanofibrillar cellulose (DA-NFC), thiolated nanofibrillar cellulose (SH-NFC), ciclooctinilated nanofibrillar cellulose (DIBCO-NFC) and aminated nanofibrillar cellulose (NH2-NFC).

14. The method of any one of claims 12 or 13, wherein the cellulose nanofibrils are covalently bounded to biological agents such as peptides, proteins, enzymes, antibodies, or nucleotide sequences to provide for a chemical and/or biological function.

15. The method of any one of claims 12 to 14, wherein the solid substrate surface is made of silicon, silicon dioxide, silicon nitride, glass, diamond, quartz, magnesium fluoride (MgF2), calcium fluoride (CaF2), ZnSe, germanium, or a polymer such as polystyrene or cellulose fibers (such as paper).

16. The method of claim 15, wherein the solid substrate surface is flat, nonporous or porous, and optionally patterned.

17. The method of claim 15 or 16, wherein the solid substrate surface is chemically modified to provide thiolated (SH); carboxylated (COOH); cholorsulfonated (SO2Cl-); aminated (NH2); alkenylated (alkenylated); and epoxidated (epoxydated) substrates.

18. The method of any of claims 12 to 17, wherein after the flow fluidic channels and reaction chambers are printed with the nanocellulose ink, the device is cured by drying, freezing or heating or any combination thereof.

19. The method of claim 18, wherein the device is cure by being dried (preferably oven dried at about 37ºC).

20. The method according to any one of claims 12 to 19, wherein the nanocellulose ink is blended with one or more binders.

21. The method according to claim 20, wherein the binder is selected from the list consisting of Poly3, Poly5 and Poly6.
